# EUROPÄISCHE PATENTSCHRIFT

(11) **EP 0 942 904 B1**
(45) Veröffentlichungstag und Bekanntmachung des Hinweises auf die Patenterteilung: **23.06.2004**
(21) Anmeldenummer: 97951934.5
(22) Anmeldetag: 21.11.1997
(51) Int. Cl.: C07D 231/44, C07D 403/04, C07D 231/38, A01N 43/56, C07D 231/18

(54) **3-THIOCARBAMOYLPYRAZOL-DERIVATE ALS PESTIZIDEN**
3-THIOCARBAMOYLPYRAZOLE DERIVATIVES AS PESTICIDES
DERIVES DE 3-THIOCARBAMOYLPYRAZOLE UTILISES COMME PESTICIDES

(30) Priorität: 04.12.1996 DE 19650197
(43) Veröffentlichungstag der Anmeldung: 22.09.1999
(73) Patentinhaber: Bayer CropScience AG, 40789 Monheim (DE)
(72) Erfinder: ALIG, Bernd, D-53639 Königswinter (DE); BERTSCH, Achim, D-51061 Köln (DE); BIELEFELDT, Dietmar, D-40883 Ratingen (DE); LUI, Norbert, D-51061 Köln (DE); MARHOLD, Albrecht, D-51373 Leverkusen (DE); ERDELEN, Christoph, D-42799 Leichlingen (DE); ANDERSCH, Wolfram, D-51469 Bergisch Gladbach (DE); TURBERG, Andreas, D-40699 Erkrath (DE); MENCKE, Norbert, D-51381 Leverkusen (DE)
(86) Internationale Anmeldenummer: PCT/EP1997/006503
(87) Internationale Veröffentlichungsnummer: WO 1998/024769

(56) Entgegenhaltungen:
- EP-A- 0 418 016
- WO-A-96/25401
- US-A- 5 629 335

## Beschreibung

Die Erfndung betrifft neue 3-Thiocarbamoylpyrazol-Derivate, mehrere Verfahren zu ihrer Herstellung und ihre Verwendung als Schädlingsbekämpfungsmittel.

Es ist bereits bekannt, dass bestimmte substituierte 1-Arylpyrazole, wie beispielsweise 5-Amino-1-[2,6-dichlor-4-(trifluormethyl)-phenyl]-3-cyano-4-[(trifluormethyl)-sulfinyl]-1H-pyrazol eine gute Wirksamkeit gegen Schädlinge besitzen (vgl. z.B. EP-A 295 117 und EP-A 352 944).

Weiterhin sind zahlreiche substituierte 1-Arylpyrazole beschrieben, die zur Bekämpfung von Schädlingen eingesetzt werden können (vgl. z.B. EP-A 201 852, EP-A 418 016 oder EP-A 0 659 745).

Die Wirkhöhe bzw. Wirkungsdauer der vorbekannten Verbindungen ist jedoch, insbesondere bei bestimmten Insekten oder bei niedrigen Anwendungskonzentrationen nicht in allen Anwendungsgebieten völlig zufriedenstellend.

Es wurden nun neue 3-Thiocarbamoylpyrazol-Derivate der allgemeinen Formel (I) gefunden, 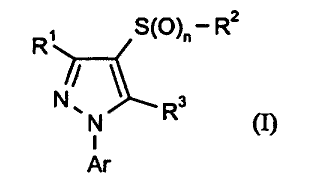 in welcher
- R¹: für H₂N-CS- steht,
- R²: für (C₁-C₆)-Halogenalkyl mit 1 bis 12 Halogenatomen; (C₂-C₆)-Halogenalkenyl mit 1 bis 8 Halogenatomen oder (C₂-C₆)-Halogenalkinyl mit 1 bis 6 Halogenatomen steht,
- R³: für Wasserstoff, Amino oder für eine der folgenden Gruppierungen steht: 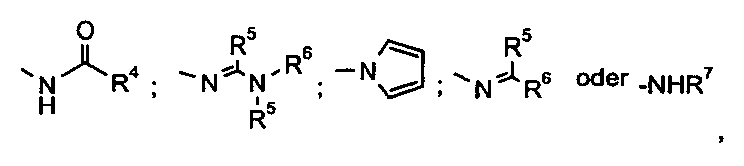 wobei
R⁴ für (C₁-C₆)-Alkyl, (C₁-C₆)-Halogenalkyl mit 1 bis 3 Halogenatomen, (C₁-C₆)-Alkoxy-(C₁-C₆)-alkyl oder für jeweils gegebenenfalls einfach bis dreifach, gleich oder verschieden durch Cyano, Nitro, Halogen, (C₁-C₆)-Alkyl, (C₁-C₆)-Alkoxy, (C₁-C₆)-Alkylthio, (C₁-C₄)-Halogenalkyl, (C₁-C₄)-Halogenalkoxy oder (C₁-C₄)-Halogenalkylthio mit jeweils 1 bis 5 Halogenatomen substituiertes Phenyl oder Pyridyl steht,
R⁵ für Wasserstoff oder (C₁-C₆)-Alkyl steht,
R⁶ für Wasserstoff, (C₁-C₆)-Alkyl oder für gegebenenfalls einfach bis dreifach, gleich oder verschieden durch Cyano, Nitro, Halogen, (C₁-C₆)-Alkyl, (C₁-C₆)-Alkoxy, (C₁-C₆)-Alkylthio, (C₁-C₄)-Halogenalkyl, (C₁-C₄)-Halogenalkoxy oder (C₁-C₄)-Halogenalkylthio mit jeweils 1 bis 5 Halogenatomen oder Hydroxy substituiertes Phenyl oder für durch Cyano, Nitro, Halogen, (C₁-C₆)-Alkyl, (C₁-C₆)-Allcoxy, (C₁-C₆)-Alkylthio, (C₁-C₄)-Halogenalkyl, (C₁-C₄)-Halogenalkoxy oder (C₁-C₄)-Halogenalkylthio mit jeweils 1 bis 5 Halogenatomen substituiertes Pyridyl steht und
R⁷ für (C₁-C₆)-Alkyl, (C₂-C₆)-Alkenyl, (C₂-C₆)-Alkinyl, Formyl, (C₁-C₆)-Alkyl-carbonyl, (C₁-C₆)-Halogenalkylcarbonyl mit 1 bis 6 Halogenatomen oder (C₁-C₆)-Alkoxycarbonyl steht,
- Ar: für jeweils gegebenenfalls einfach bis dreifach, gleich oder verschieden durch Halogen, (C₁-C₆)-Halogenalkyl, (C₁-C₆)-Halogenalkylthio, (C₁-C₆)-Halogenalkoxy, (C₁-C₆)-Alkoxy, Hydrazino, (C₁-C₆)-Dialkylhydrazino, Amino, -(C₁-C₆)-Alkylamino, Di(C₁-C₆)-alkylamino, (C₁-C₆)-Alkylimino, Cyano, -(C₁-C₆)-Alkylthio oder die Gruppierung 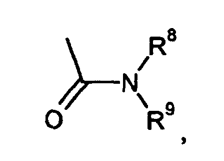 worin
R⁸ und R⁹ gleich oder verschieden sind und für Wasserstoff oder (C₁-C₆)-Alkyl stehen, substituiertes Phenyl oder Pyridyl steht und
- n: für eine Zahl 0, 1 oder 2 steht.

Weiterhin wurde gefunden, daß man die neuen 3-Thiocarbamoylpyrazol-Derivate der Formel (I) erhält, wenn man
a) 3-Cyanopyrazol-Derivate der Formel (II) 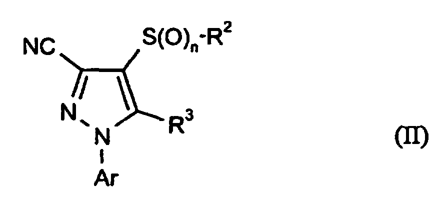 in welcher
   - Ar, R², R³ und n: die oben angegebene Bedeutung haben,
   mit Schwefelwasserstoff, gegebenenfalls in Gegenwart eines Reaktionshilfsmittels und gegebenenfalls in Gegenwart eines Verdünnungsmittels umsetzt;
   oder
b) 3-Thiocarbamoylpyrazol-Derivate der Formel (III) 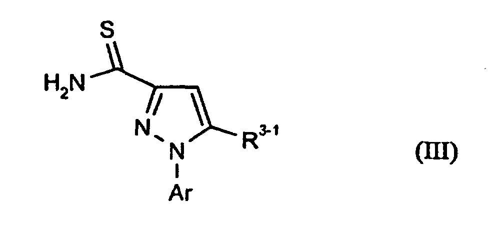 in welcher
   - Ar: die oben angegebene Bedeutung hat und
   - R³⁻¹: für eine der folgenden Gruppierungen steht: 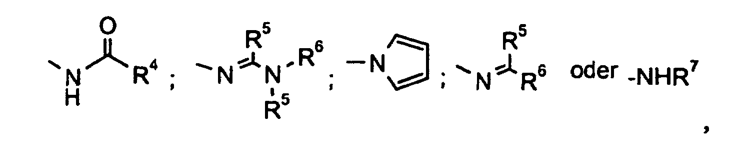
   wobei
   - R4, R⁵, R⁶ und R⁷: die oben angegebene Bedeutung haben,
   mit Sulfenylhalogeniden der Formel (IV)

   Hal-S-R² (IV)

   in welcher
   - R²: die oben angegebene Bedeutung hat und
   - Hal: für Halogen, insbesondere Chlor oder Brom steht,
   gegebenenfalls in Gegenwart eines Verdünnungsmittels und gegebenenfalls in Gegenwart eines Reaktionshilfsmittels umsetzt;
   oder
c) die gemäß den Verfahren (a) oder (b) erhältlichen 2-Thiocarbamoylpyrazol Derivate der Formel (Ia) 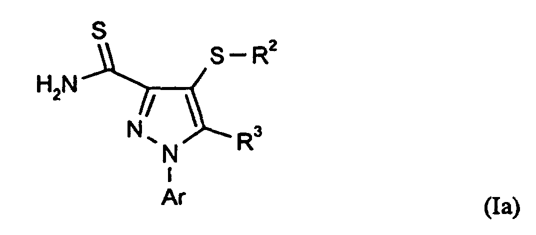
in welcher
- Ar, R² und R³: die oben angegebene Bedeutung haben,
mit Oxidationsmitteln gegebenenfalls in Gegenwart eines Verdünnungsmittels und gegebenenfalls in Gegenwart eines Katalysators oxidiert.

Schließlich wurde gefunden, daß die neuen 3-Thiocarbamoylpyrazol-Derivate der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- R²: steht bevorzugt für (C₁-C₄)-Halogenalkyl mit 1 bis 9 gleichen oder verschiedenen Halogenatomen aus der Reihe Fluor, Chlor und Brom, (C₂-C₄)-Halogenalkenyl mit 1 bis 5 gleichen oder verschiedenen Halogenatomen aus der Reihe Fluor, Chlor und Brom oder (C₂-C₄)-Halogenalkinyl mit 1 bis 5 gleichen oder verschiedenen Halogenatomen aus der Reihe Fluor, Chlor und Brom.
- R³: steht bevorzugt für Wasserstoff, Amino oder für eine der folgenden Gruppierungen: 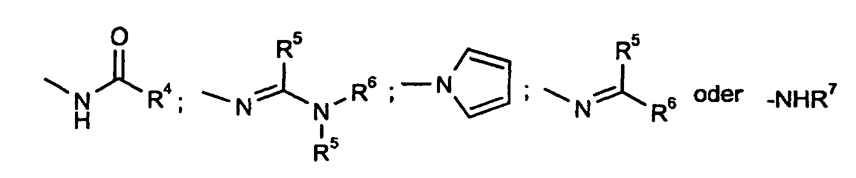 wobei
R⁴ für (C₁-C₄)-Alkyl, (C₁-C₄)-Halogenalkyl mit 1-3 Halogenatomen, (C₁-C₄)-Alkoxy-(C₁-C₂)-alkyl oder für gegebenenfalls einfach bis dreifach gleich oder verschieden durch Hydroxy, Cyano, Nitro, Halogen, C₁-C₄-Alkyl, C₁-C₄-Alkoxy, C₁-C₂-Halogenalkyl, C₁-C₂-Halogenalkoxy oder C₁-C₂-Halogenalkylthio mit jeweils 1 bis 3 Halogenatomen substituiertes Phenyl steht,
R⁵ für Wasserstoff oder (C₁-C₄)-Alkyl steht,
R⁶ für Wasserstoff, (C₁-C₄)-Alkyl oder für gegebenenfalls einfach oder zweifach, gleich oder verschieden durch Hydroxy, Cyano, Nitro, Halogen, C₁-C₄-Alkyl, C₁-C₄-Alkoxy, C₁-C₂-Halogenalkyl, C₁-C₂-Halogenalkoxy oder C₁-C₂-Halogenalkylthio mit jeweils 1 bis 3 Halogenatomen substituiertes Phenyl, insbesondere für 4-Hydroxy-3-methoxy-phenyl steht und
R⁷ für (C₁-C₄)-Alkyl, (C₂-C₄)-Alkenyl, (C₂-C₄)-Alkinyl, Formyl, (C₁-C₄)-Alkyl-carbonyl, (C₁-C₄)-Halogenalkyl-carbonyl mit 1 bis 5 gleichen oder verschiedenen Halogenatomen aus der Reihe Fluor, Chlor oder Brom oder (C₁-C₄)-Alkoxycarbonyl steht.
- Ar: steht bevorzugt für jeweils gegebenenfalls einfach bis dreifach, gleich oder verschieden durch Fluor, Chlor, Trifluormethyl, Trifluormethylthio, Trifluormethoxy, Methoxy, Hydrazino, Dimethylhydrazino, Amino, Methylamino, Dimethylamino, Iminomethyl, Cyano, Methylthio oder die Gruppierung 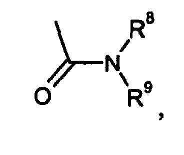 worin
R⁸ und R⁹ gleich oder verschieden sind und für Wasserstoff oder (C₁-C₄)-Alkyl stehen, substituiertes Phenyl oder Pyridyl.
- R²: steht besonders bevorzugt für einen der Reste:

-CF₃, -CHF₂

-CF₂-CH₃, -CF₂-CHF₂, -CF₂-CHFCl,

-CH₂-CF₃, -CH₂-CF₂Cl,

-CH₂-CF₂-CHF₂,

-CF₂-CFCl-CF₃,

-C(Cl)(CF₃)-CF₂Cl, -C(Cl)(CF₃)-CHCl-CF₃,

-C(CF3)=CCl₂
- R³: steht besonders bevorzugt für Wasserstoff, Amino oder eine der Gruppierungen:

-NH-CO-CH₃, -NH-CO-C₂H₅,

-N=CH-NH₂, -N=C(CH₃)-NH₂,

-N=CH-N(CH₃)₂, -N=C(CH₃)-N(CH₃)₂,

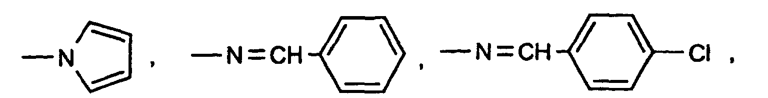

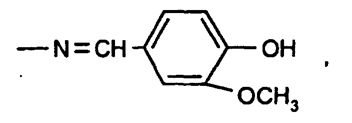

-NHC₂H₅ oder -NH-CH₂-CH=CH₂.
- Ar: steht besonders bevorzugt für
(1) zweifach oder dreifach, gleich oder verschieden substituiertes Phenyl, wobei in der 2-Position Fluor oder Chlor, in der 4-Position Trifluormethyl und in der 6-Position Fluor, Chlor, Cyano, Methoxy, Methylthio, Trifluormethyl, Trifluormethoxy, Trifluormethylthio oder Hydrazino stehen; oder
(2) einen 2-Pyridyl-Rest, welcher in der 4-Position durch Trifluormethyl und in der 6-Position durch Fluor oder Chlor substituiert ist.

Die oben aufgeführten oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

In den oben und nachstehend aufgeführten Restedefinitionen sind Kohlenwasserstoffreste, wie Alkyl oder Alkenyl - auch in Verbindung mit Heteroatomen wie Alkoxy oder Alkylthio - soweit möglich jeweils geradkettig oder verzweigt.

Bevorzugt sind Verbindungen der Formel (IA) 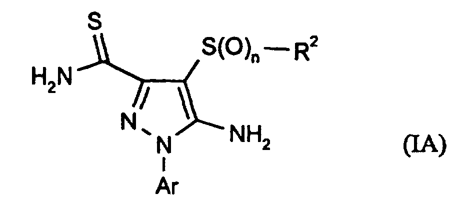 in welcher
- Ar, R² und n: die oben genannte Bedeutung haben.

Beispiele für die neuen 3-Thiocarbamoylpyrazol-Derivate sind in den Tabellen 1 bis 60 aufgeführt:

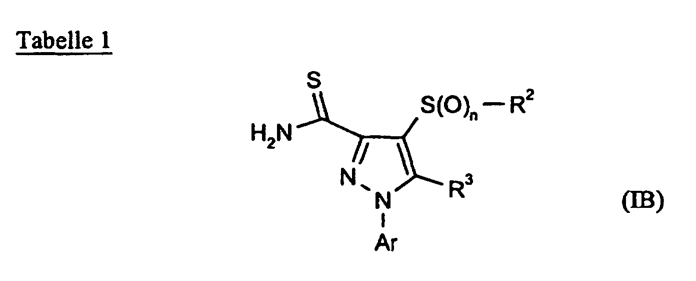

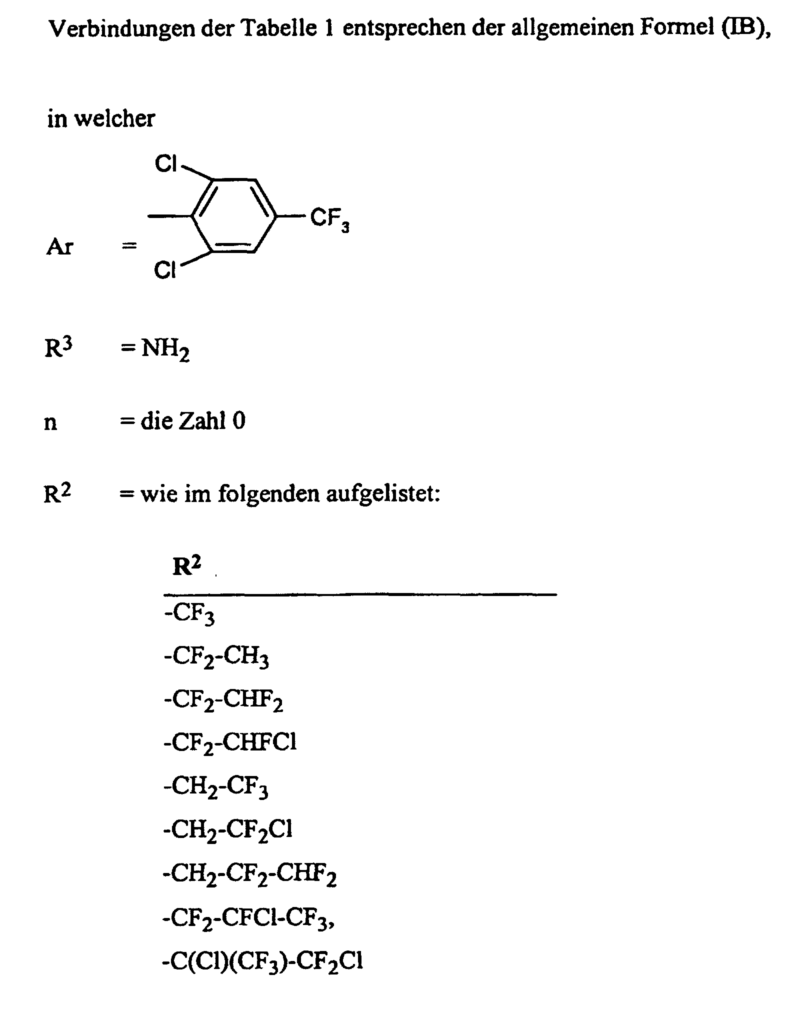

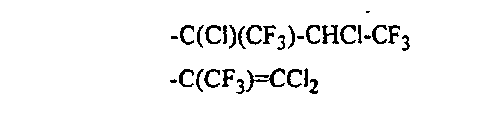

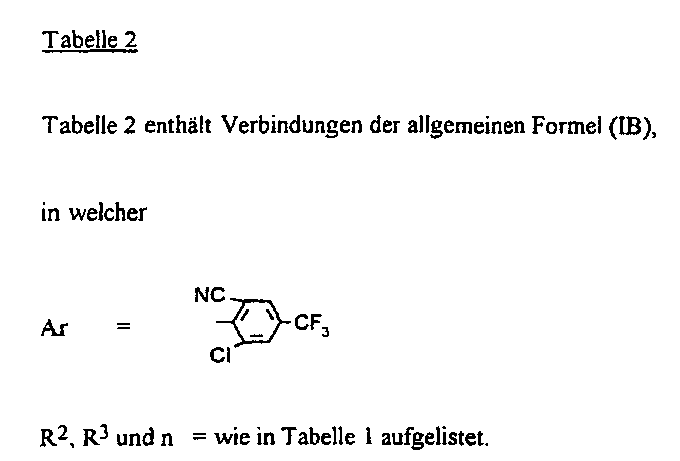

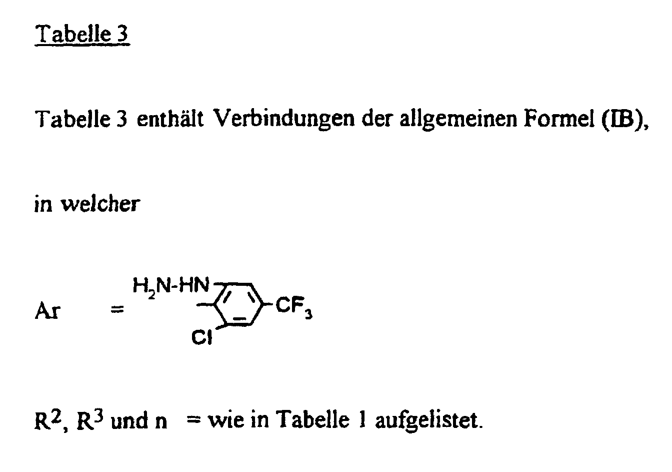

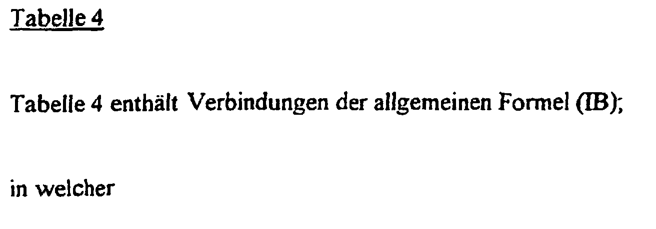

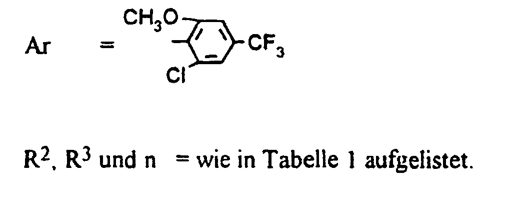

**Tabellen 5 bis 8**

| |
|---|
| Tabellen 5 bis 8 enthalten Verbindungen der allgemeinen Formel (IB), |
| in welcher |
| R³ = H |
| Ar, R² und n = wie in den Tabellen 1 bis 4 aufgelistet. |

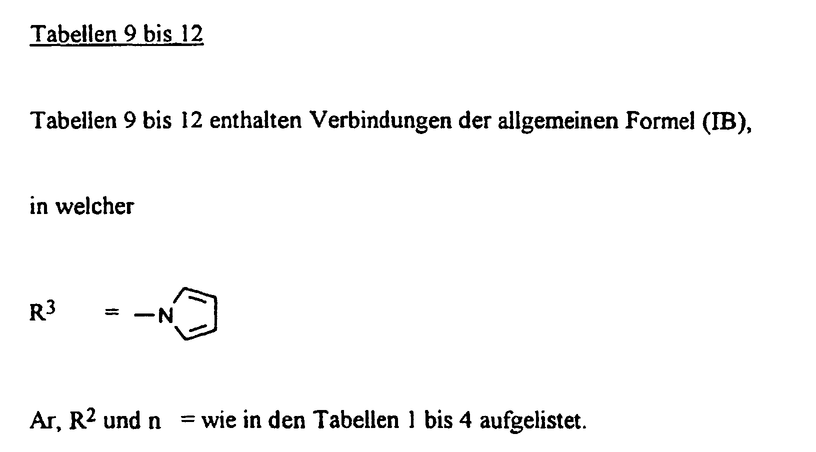

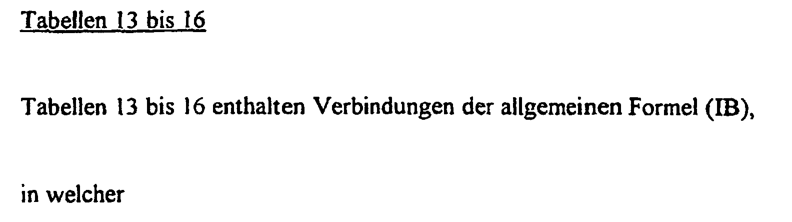

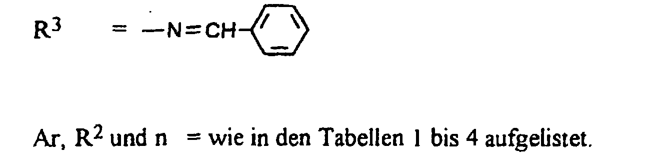

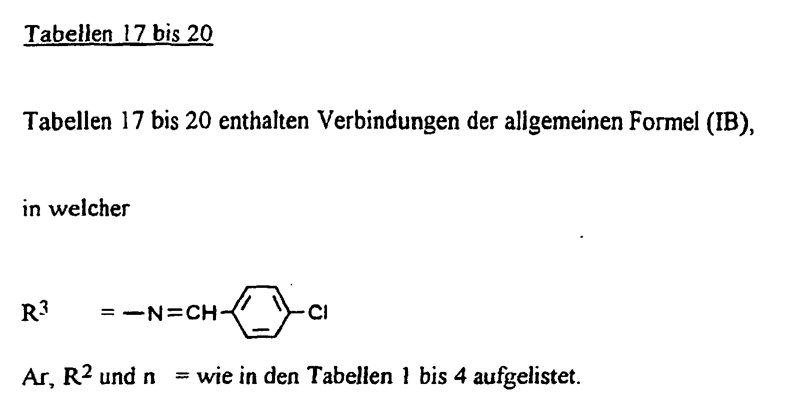

**Tabellen 21 bis 40**

| |
|---|
| Tabellen 21 bis 40 enthalten Verbindungen der allgemeinen Formel (IB), |
| in welcher |
| n = die Zahl 1 |
| Ar, R² und R³ = wie in den Tabellen 1 bis 20 aufgelistet. |

**Tabellen 41 bis 60**

| |
|---|
| Tabellen 41 bis 60 enthalten Verbindungen der allgemeinen Formel (IB), |
| in welcher |
| n = die Zahl 2 |
| Ar, R² und R³ = wie in den Tabellen 1 bis 20 aufgelistet. |

Verwendet man beispielsweise 5-Amino-3-cyano-4-(1,1-difluorethylthio)-1-(2,6-dichlor-4-trifluormethylphenyl)-pyrazol und Schwefelwasserstoff als Ausgangsstoffe, so kann der Reaktionsablauf des erfindungsgemäßen Verfahrens (a) durch das folgende Formelschema wiedergegeben werden:

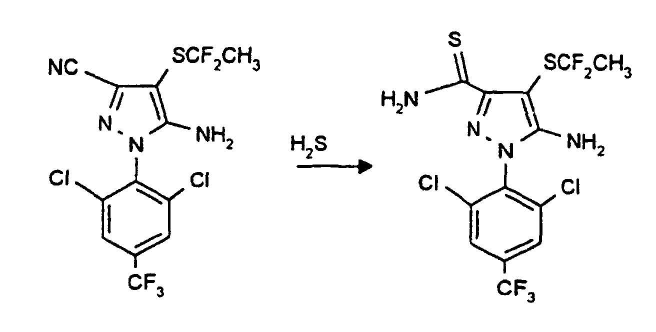

Verwendet man beispielsweise 1-(2,6-Dichlor-4-trifluormethylphenyl)-3-thiocarbamoyl-5-(pyrrol-1-yl)-pyrazol und 1,1-Difluorethylsulfensäurechlorid als Ausgangsstoffe, so kann der Reaktionsablauf des erfindungsgemäßen Verfahrens (b) durch das folgende Formelschema wiedergegeben werden:

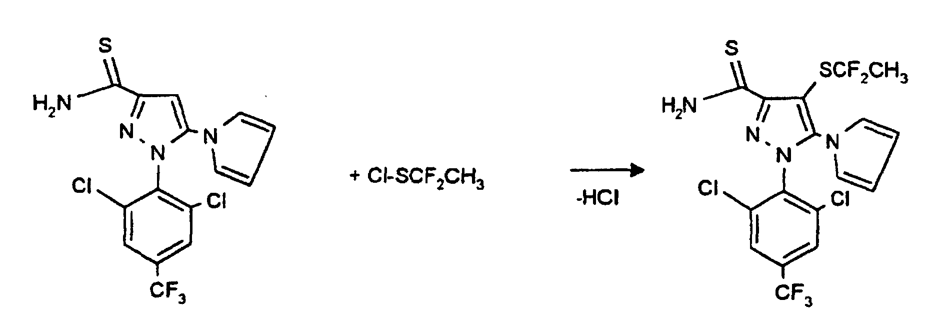

Verwendet man beispielsweise 5-(Pyrrol-1-yl)-3-thiocarbamoyl-4-(1,1-difluorethylthio)-1-(2,6-dichlor-4-trifluormethyl-phenyl)-pyrazol und Schwefelwasserstoff als Ausgangsstoffe, so kann der Reaktionsablauf des erfindungsgemäßen Verfahrens (c) durch das folgende Formelschema wiedergegeben werden:

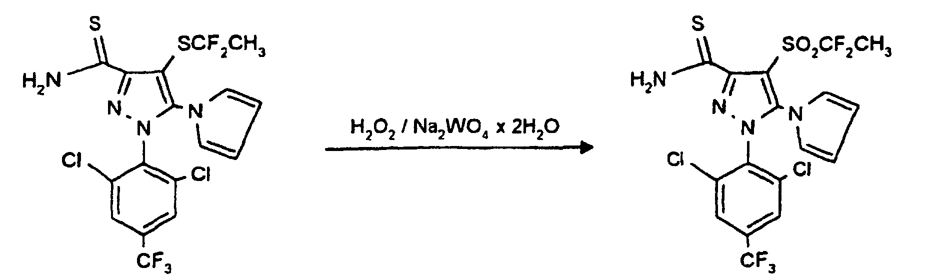

Die zur Durchführung des erfindungsgemäßen Verfahrens (a) als Ausgangsstoffe zu verwendenden 3-Cyanopyrazol-Derivate der Formel (II) sind bekannt (vgl. z.B. EP-A 0 295 117 und EP-A 0 659 745) und/oder können in Analogie zu bekannten Verfahren hergestellt werden.

Die zur Durchführung des erfindungsgemäßen Verfahrens (b) als Ausgangsstoffe zu verwendenden 3-Thiocarbamoylpyrazol-Derivate der Formel (III) sind neu und ebenfalls Gegenstand der Erfindung.

Die Verbindungen der Formel (III) können erhalten werden, indem man 2-Cyanopyrazole der Formel (V) 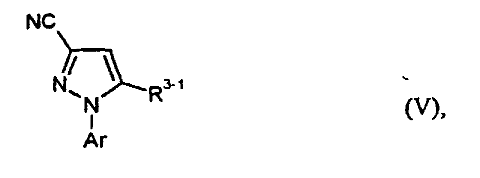 in welcher
- Ar und R³⁻¹: die oben angegebene Bedeutung haben,
gemäß dem erfindungsgemäßen Verfahren (a) mit Schwefelwasserstoff, gegebenenfalls in Gegenwart eines Reaktionshilfsmittels und gegebenenfalls in Gegenwart eines Verdünnungsmittels umsetzt.

Die 3-Cyanopyrazole der Formel (V) sind bekannt und/oder können in bekannter Art und Weise erhalten werden, indem man z.B. die entsprechenden 5-Amino-3-cyanopyrazole der Formel (VI) 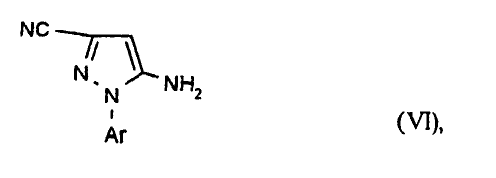 in welcher
- Ar: die oben angegebene Bedeutung hat,
in üblicher Art und Weise an der Aminogruppe derivatisiert (vgl. z.B. EP-A 0 659 745).

Die Verbindungen der Formel (III) können auch erhalten werden, indem man 5-Amino-3-thiocarbamoylpyrazole der Formel (VII) 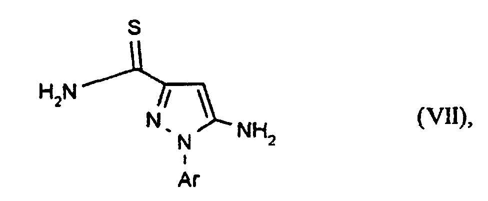 in welcher
- Ar: die oben angegebene Bedeutung hat,
in üblicher Art und Weise an der Aminogruppe derivatisiert (vgl. z.B. EP-A 0 659 745).

Die 5-Amino-3-thiocarbamoylpyrazole der Formel (VII) sind neu und ebenfalls Gegenstand der Erfindung.

Sie werden erhalten, indem man 5-Amino-3-cyanopyrazole der Formel (VI) gemäß dem erfindungsgemäßen Verfahren (a) mit Schwefelwasserstoff gegebenenfalls in Gegenwart eines Verdünnungsmittels umsetzt (vgl. auch die Herstellungsbeispiele).

Die außerdem für das erfindungsgemäße Verfahren (b) als Ausgangsstoffe zu verwendenden Sulfenylhalogenide der Formel (IV) sind allgemein bekannte Verbindungen der organischen Chemie.

Die für das erfindungsgemäße Verfahren (c) als Ausgangsstoffe zu verwendenden 3-Thiocarbamoylpyrazol-Derivate der Formel (Ia) sind erfindungsgemäße Verbindungen.

Das erfindungsgemäße Verfahren (a) wird vorzugsweise unter Verwendung eines Verdünnungsmittels durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- oder Methyl-isobutyl-keton, Ester wie Essigsäuremethylester oder -ethylester, Nitrile wie z.B. Acetonitril oder Propionitril, Amide wie z.B. Dimethylformamid, Dimethylacetamid und N-Methylpyrrolidon sowie Dimethylsulfoxid, Tetramethylensulfon oder Hexamethylenphosphorsäuretriamid.

Als Reaktionshilfsmittel können bei dem erfindungsgemäßen Verfahren (a) alle üblicherweise für derartige Umsetzungen verwendbaren Basen eingesetzt werden. Vorzugsweise infrage kommen basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethylanilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) oder 1,4-Diazabicyclo-[2,2,2]-octan (DABCO). Es ist auch möglich, ein im Überschuß eingesetztes Reaktionshilfsmittel als Verdünnungsmittel zu verwenden.

Die Reaktionstemperaturen können bei dem erfindungsgemäßen Verfahren (a) in einem größeren Bereich variiert werden. Im allgemeinen arbeitet man bei Temperaturen zwischen 0°C und 100°C, vorzugsweise bei Temperaturen zwischen 10°C und 80°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) wird der Schwefelwasserstoff in der Regel im Überschuß eingesetzt. Die Reaktionen werden im allgemeinen in einem geeigneten Verdünnungsmittel in Gegenwart einer basischen Stickstoffverbindung durchgeführt. Die Aufarbeitung erfolgt nach üblichen Methoden (vgl. die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril; Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Säuren, wie beispielsweise Essigsäure.

Das erfindungsgemäße Verfahren (b) kann gegebenenfalls in Gegenwart eines Reaktionshilfsmittels durchgeführt werden. Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate oder Hydrogen-carbonate, wie Natriumcarbonat, Kaliumcarbonat oder Natriumhydrogen-carbonat sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +120°C, vorzugsweise bei Temperaturen zwischen 0°C und +50°C.

Zur Durchführung des erfindungsgemäßen Verfahrens (b) setzt man pro Mol in an 4-Stellung substituiertem 1-Arylpyrazol der Formel (III) im allgemeinen 1,0 bis 2,5 Mol, vorzugsweise 1,0 bis 1,5 Mol an Sulfenylhalogenid der Formel (IV) und gegebenenfalls 1,0 bis 2,5 Mol, vorzugsweise 1,0 bis 1,5 Mol an Reaktionshilfsmittel ein. Die Reaktionsführung, Aufarbeitung und Isolierung der Reaktionsprodukte erfolgt nach allgemein üblichen Verfahren.

Als Oxidationsmittel zur Durchführung des erfindungsgemäßen Verfahrens (c) kommen alle üblichen zur Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Als Verdünnungsmittel zur Durchführung des erfindungsgemäßen Verfahrens (c) kommen ebenfalls inerte organische Lösungsmittel infrage. Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolare aprotische Lösungsmittel, wie Acetonitril, Aceton, Essigsäureethylester oder Dimethylformamid.

Das erfindungsgemäße Verfahren (c) kann gegebenenfalls in Gegenwart eines Säurebindemittels durchgeführt werden. Als solche kommen alle üblicherweise verwendbaren organischen und anorganischen Säurebindemittel infrage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (c) kann gegebenenfalls in Gegenwart eines geeigneten Katalysators durchgeführt werden. Als solche kommen alle üblicherweise für derartige Schwefeloxidationen gebräuchlichen Metallsalz-Katalysatoren infrage. Beispielhaft genannt seien Ammoniummolybdat und Natriumwolframat.

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +70°C, vorzugsweise bei Temperaturen zwischen 0°C und +50°C.

Zur Durchführung des erfindungsgemäßen Verfahrens (c) setzt man pro Mol an Verbindung der Formel (Ia) im allgemeinen 0,8 bis 1,2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an Verbindung der Formel (Ia) im allgemeinen 1,8 bis 3,0 Mol, vorzugsweise doppelt molare Mengen an Oxidationsmittel ein. Die Reaktionsdurchführung, Aufarbeitung und Isolierung der Endprodukte erfolgt nach üblichen Verfahren.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (I) zeichnen sich insbesondere durch eine hohe insektizide Wirksamkeit mit teilweise wurzelsystemischen Eigenschaften aus.

Sie lassen sich mit besonders gutem Erfolg zur Bekämpfung von pflanzenschädigenden Insekten, wie beispielsweise gegen die Meerrettichblattkäferlarven (Phaedon cochlaeriae), die Raupen der Kohlschabe (Plutella maculipennis), die grüne Reiszikade (Nephotettix cincticeps), die Raupen des Eulenfalters (Spodoptera frugiperda), die Pfirsichblattläuse (Mycus persicae) oder die Larven des Gurkenkäfers (Diabrotica balteata) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(otolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,

### Guazatine,

Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine gute Wirkung gegen Fliegen (Musca domestica), entwicklungshemmende Wirkung gegen Fliegenlarven von Lucilia cuprina sowie eine gute Wirkung gegen Schaben (Periplaneta americana) und gegen Zecken (Boophilus microplus), auch in Form einer Hemmung der Eiablage.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus
Hautflügler wie
Sirex juvencus, Urocerus gigas, Urocerus gigas taignus, Urocerus augur
Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze, wie Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsaureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

### Herstellungsbeispiele

### Beispiel 1

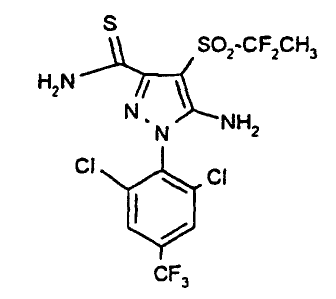

11 g (0,024 Mol) 5-Amino-3-cyano-4-(1,1-difluorethylsulfonyl)-1-(2,6-dichlor-4-trifluormethylphenyl)-pyrazol werden in 80 ml Pyridin und 10 ml Triethylamin gelöst. Danach wird bei Raumtemperatur ca. 3 Stunden lang Schwefelwasserstoff eingeleitet. Die Reaktionslösung wird anschließend mit Wasser versetzt und mehrmals mit Dichlormethan extrahiert. Nach dem Trocknen über Magnesiumsulfat wird durch Abdestillieren des Lösungsmittels im Vakuum eingeengt, der ölige Rückstand in Diethylether verrührt und abgesaugt.

Man erhält 8 g (68 % der Theorie) 5-Amino-4-(1,1-difluorethylsulfonyl)-1-(2,6-dichlor-4-trifluormethylphenyl)-3-thiocarbamoyl-pyrazol vom Schmelzpunkt 228-29°C.

Analog Beispiel 1 bzw. gemäß den allgemeinen Angaben zur Herstellung werden die in der folgenden Tabelle A angegebenen Verbindungen der Formel (I) erhalten:

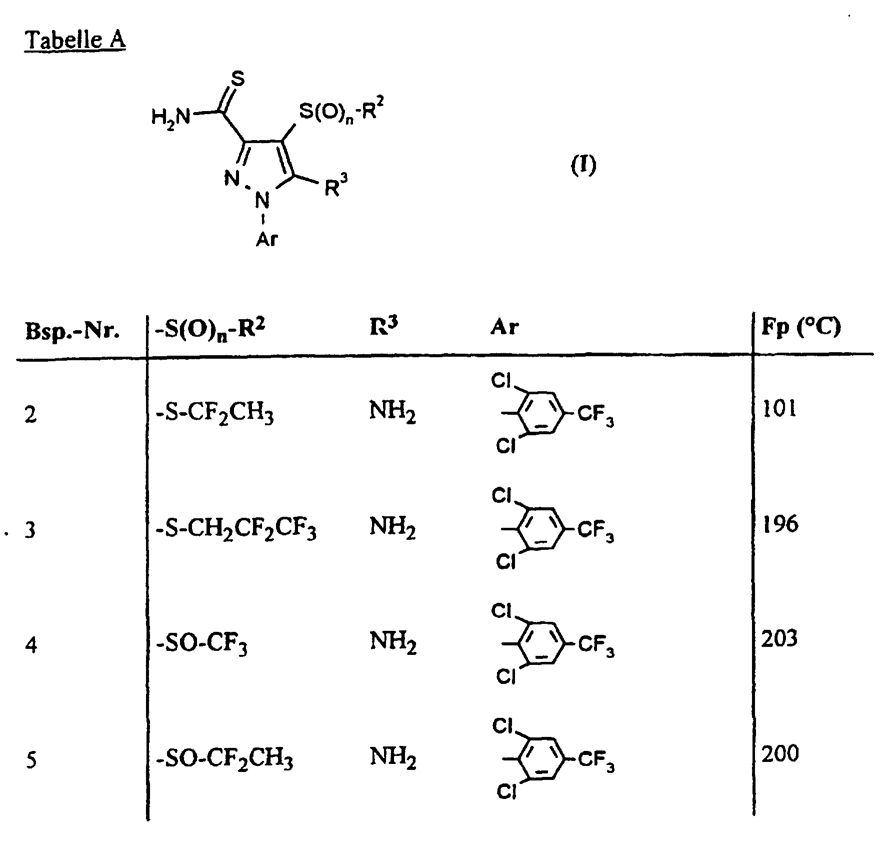

### Herstellung der neuen Ausgangsprodukte der Formel (VII)

Beispiel (VII-1)

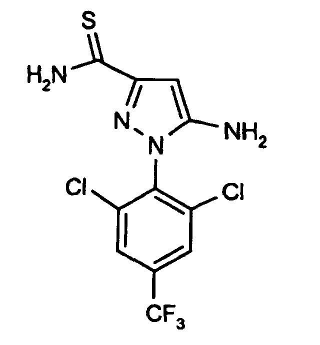

3,8 g (0,012 Mol) 5-Amino-3-Cyano-1-(2,6-dichlor-4-trifluormethylphenyl)-pyrazol werden in 50 ml Pyridin und 5 ml Triethylamin gelöst. Danach wird bei Raumtemperatur ca. 2 Stunden lang Schwefelwasserstoff eingeleitet, anschließend wird noch 10 Minuten bei 50°C nachgerührt. Die Reaktionslösung wird im Vakuum eingeengt, der verbleibende Rückstand mit Wasser und Dichlormethan versetzt. Man extrahiert mehrmals mit Dichlormethan, trocknet die vereinigten Dichlormethan-Phasen über Magnesiumsulfat und engt im Vakuum ein.

Man erhält 3,4 g (81 % der Theorie) 5-Amino-1-(2,6-dichlor-4-trifluormethylphenyl)-3-thiocarbamoyl-pyrazol.
¹H-NMR (in DMSO mit TMS als innerem Standard; δ in ppm):
9,49 (1H); 9,08 (1H); 8,21 (2H); 5,96 (1H); 5,69 (2H).

### Herstellung der neuen Ausgangsprodukte der Formel (III)

Beispiel (III-1)

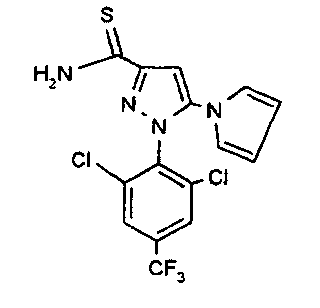

Beispiel (III-I) wird, ausgehend von 3-Cyano-1-(2,6-dichlor-4-trifluormethylphenyl)-5-(pyrrol-1-yl)-pyrazol, analog Beispiel (VII-I) erhalten.
¹H-NMR (in DMSO mit TMS als innerem Standard; δ in ppm):
9,95 (1H); 9,58 (1H); 8,30 (2H); 7,19 (1H); 6,20 (2H); 6,13 (2H).

### Anwendungsbeispiele

In nachfolgenden Anwendungsbeispielen werden nachstehend aufgeführte Verbindungen als Vergleichssubstanzen eingesetzt:

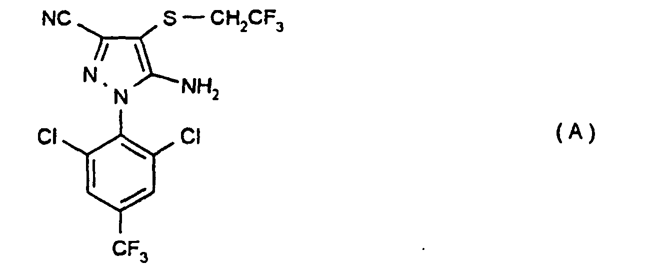

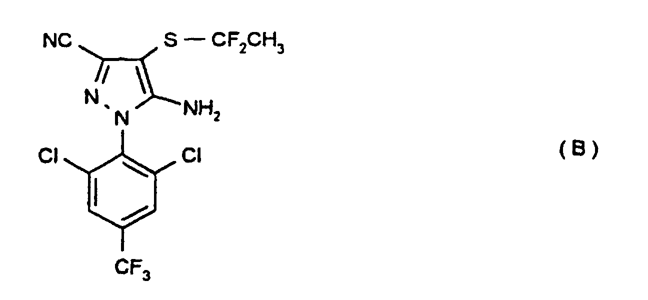

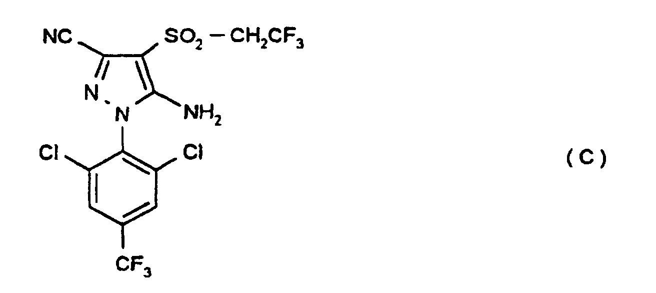

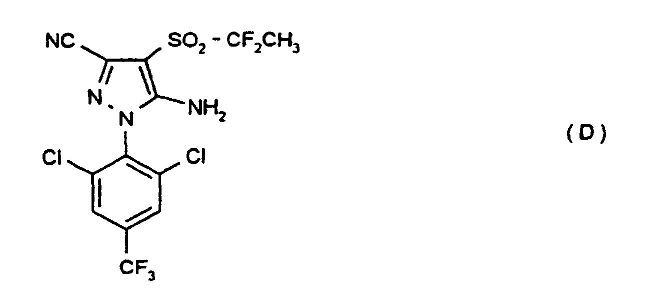

### (Alle Verbindungen bekannt aus EP-A 0 659 745)

### Beispiel A

| **Phaedon-Larven-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test bewirkten bei einer beispielhaften Wirkstoffkonzentration von 0,00001 % z.B. die Verbindungen der Herstellungsbeispiele 1 und 2 eine Abtötung von 100 % jeweils nach 3 Tagen, während die bekannte Verbindung (A) lediglich 25 % Abtötung zeigte.

### Beispiel B

| **Plutella-Test** | | |
|---|---|---|
| Lösungsmittel | 7 Gewichtsteile | Dimethylfomamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test bewirkten bei einer beispielhaften Wirkstoffkonzentration von 0,0001 % z.B. die Verbindung des Herstellungsbeispiels 1 eine Abtötung von 75 % und die Verbindung des Herstellungsbeispiels 2 eine Abtötung von 100 % jeweils nach 3 Tagen, während die bekannte Verbindung (A) lediglich 15 % Abtötung zeigte.

### Beispiel C

| **Spodoptera frugiperda-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test bewirkten bei einer beispielhaften Wirkstoffkonzentration von 0,01 % z.B. die Verbindungen der Herstellungsbeispiele 1, 2 und 4 eine Abtötung von 100 % jeweils nach 7 Tagen, während die bekannten Verbindungen (B) lediglich 10 % und (C) keine Abtötung zeigten.

### Beispiel D

| **Nephotettix-Test** | |
|---|---|
| Lösungsmittel | 20 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test bewirkten bei einer beispielhaften Wirkstoffkonzentration von 0,1 % z.B. die Verbindung der Herstellungsbeispiele 2 und 4 eine Abtötung von 100 % und die Verbindung des Herstellungsbeispiels 5 eine Abtötung von 80 % jeweils nach 6 Tagen, während die bekannten Verbindungen (A) lediglich 10 % und (D) keine Abtötung zeigten.

### Beispiel E

| **Myzus-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test bewirkten bei einer beispielhaften Wirkstoffkonzentration von 0,1 % z.B. die Verbindungen folgender Herstellungsbeispiele folgende Abtötungen:

1 = 80 %; 2 = 98 % und 4 = 100 %; jeweils nach 6 Tagen, während die bekannten Verbindungen (C) lediglich 50 % und (B) keine Wirkung zeigten.

### Beispiel F

| **Grenzkonzentrationstest / Bodeninsekten** | |
|---|---|
| Testinsekt | **Diabrotica balteata - Larven im Boden** |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration. Dabei spielt die Konzentration des Wirkstoffes in der Zubereitung praktisch keine Rolle, entscheidend ist allein die Wirkstoffgewichtsmenge pro Volumeneinheit Boden, welche in ppm (mg/l) angegeben wird. Man füllt den Boden in 0.5 l Töpfe und läßt diese bei 20°C stehen.

Sofort nach dem Ansatz werden je Topf 5 vorgekeimte Maiskörner gelegt. Nach 1 Tag werden die Testinsekten in den behandelten Boden gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test bewirkten bei einer beispielhaften Wirkstoffkonzentration von 0,002 % z.B. die Verbindungen der Herstellungsbeispiele 2, 4 und 5 eine Abtötung von 100 %.

### Beispiel G

| **Grenzkonzentrationstest / Wurzelsystemische Wirkung** | |
|---|---|
| Testinsekt | **Phaedon cochleariae-Larven** |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test bewirkten bei einer beispielhaften Wirkstoffkonzentration von 0,002 % z.B. die Verbindungen der Herstellungsbeispiele 1, 4 und 5 eine Abtötung von 100 %.

### Beispiel H

| **Test mit Fliegen (Musca domestica)** | |
|---|---|
| Testtiere | adulte Musca domestica, Stamm Reichswald (OP, SP, Carbamat-resistent) |
| Lösungsmittel | 35 Gewichtsteile Ethylenglykolmonomethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (φ 9,5 cm) pipettiert, die sich in Petrischalen entsprechender Größe befinden. Nach Trocknung der Filterscheiben werden 25 Testtiere in die Petrischale überführt und abgedeckt.

Nach 1, 3, 5 und 24 Stunden wird die Wirksamkeit der Wirkstoffzubereitung ermittelt. Dabei bedeutet 100%, daß alle Fliegen abgetötet wurden; 0% bedeutet, daß keine Fliegen abgetötet wurden.

Bei diesem Test zeigten z.B. die Verbindungen der Herstellungsbeispiele 1, 2, 4 und 5 bei einer beispielhaften Wirkstoffkonzentration von 100 ppm eine 100 %-ige Wirkung.

### Beispiel I

| **Test mit Fliegenlarven / Entwicklungshemmende Wirkung** | |
|---|---|
| Testtiere | Alle larvalen Stadien von Lucilia cuprina (OP-resistent) |
| | [Puppen und Adulte (ohne Kontakt zum Wirkstoff)] |
| Lösungsmittel | 35 Gewichtsteile Ethylenglykolmonomethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

30 - 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ±1,5°C, 70 % rel. Feuchte ±10 %) aufbewahrt. Die Wirkungskontrolle erfolgt nach 24 Stunden und 48 Stunden (larvizide Wirkung). Nach dem Auswandern der Larven (ca. 72 Stunden) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 Stunden (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Fliegenentwicklung, bzw. ein Entwicklungsstillstand vor dem Adulten-Stadium. Dabei bedeutet 100 % larvizide Wirkung, dass nach 48 Stunden alle Larven abgestorben sind. 100 % entwicklungsinhibitorische Wirkung bedeutet, daß keine adulte Fliegen geschlüpft sind.

Bei diesem Test zeigten z.B. die Verbindungen gemäß den Herstellungsbeispielen 1, 2, 4 und 5 bei einer beispielhaften Wirkstoffkonzentration von 100 ppm eine 100 %-ige Wirkung.

### Beispiel J

| **Test mit Boophilus microplus resistent/SP-resistenter Parkhurst-Stamm** | |
|---|---|
| Testtiere | adulte gesogene Weibchen |
| Lösungsmittel | 35 Gewichtsteile Ethylenglykolmonomethylether |
| | 35 Gewichtsteile Nonylphenolpolyglykolether |

Zur Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Dabei bedeutet 100 %, daß alle Zecken abgetötet wurden; 0 % bedeutet, daß keine Zecken abgetötet wurden.

Bei diesem Test zeigten z.B. die Verbindungen der Herstellungsbeispiele 2, 4 und 5 bei einer beispielhaften Wirkstoffkonzentration von 100 ppm eine 100 %-ige Wirkung.

### Beispiel K

| **Test mit Boophilus microplus resistent/SP-resistenter Parkhurst-Stamm** | |
|---|---|
| Testtiere | Adulte gesogene Weibchen |
| Lösungsmittel | Dimethylsulfoxid |

20 mg Wirkstoff werden in 1 ml Dimethylsulfoxid gelöst, geringere Konzentrationen werden durch Verdünnen in dem gleichen Lösungsmittel hergestellt.

Der Test wird in 5-fach-Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

Bei diesem Test zeigten z.B. die Verbindungen der Herstellungsbeispiele 1, 3, 4 und 5 bei einer beispielhaften Wirkstoffkonzentration von 20 µg/Tier eine 100 %-ige Wirkung.

### Beispieil L

| **Schabentest** | |
|---|---|
| Testtiere | Periplaneta americana |
| Lösungsmittel | 35 Gewichtsteile Ethylenglykolmonomethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (φ 9,5 cm) pipettiert, die sich in Petrischalen entsprechender Größe befinden. Nach Trocknung der Filterscheiben werden 5 Testtiere Periplaneta americana überführt und abgedeckt.

Nach 3 Tagen wird die Wirksamkeit der Wirkstoffzubereitung bestimmt. Dabei bedeutet 100%, daß alle Schaben abgetötet wurden; 0% bedeutet, daß keine Schaben abgetötet wurden.

In diesem Test zeigten z.B. die Verbindungen der Herstellungsbeispiele 1 und 2 bei einer beispielhaften Wirkstoffkonzentration von 100 ppm eine 100 %-ige Wirkung.

## Patentansprüche

1. Verbindungen der allgemeinen Formel (I) 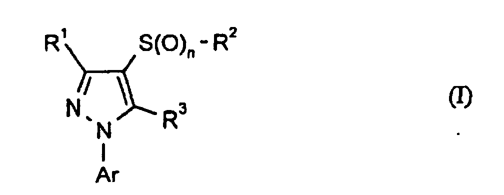 in welcher
R¹ für H₂N-CS- steht,
R² für (C₁-C₆)-Halogenalkyl mit 1 bis 12 Halogenatomen; (C₂-C₆)-Halogenalkenyl mit 1 bis 8 Halogenatomen oder (C₂-C₆)-Halogenalkinyl mit 1 bis 6 Halogenatomen steht,
R³ für Wasserstoff, Amino oder für eine der folgenden Gruppierungen steht: 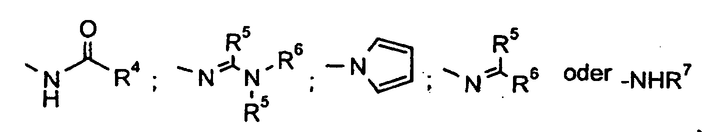 wobei
R⁴ für (C₁-C₆)-Alkyl, (C₁-C₆)-Halogenalkyl mit 1 bis 3 Halogenatomen, (C₁-C₆)-Alkoxy-(C₁-C₆)-alkyl oder für jeweils gegebenenfalls einfach bis dreifach, gleich oder verschieden durch Cyano, Nitro, Halogen, (C₁-C₆)-Alkyl, (C₁-C₆)-Alkoxy, (C₁-C₆)-Alkylthio, (C₁-C₄)-Halogenalkyl, (C₁-C₄)-Halogenalkoxy oder (C₁-C₄)-Halogenalkylthio mit jeweils 1 bis 5 Halogenatomen substituiertes Phenyl oder Pyridyl steht,
R⁵ für Wasserstoff oder (C₁-C₆)-Alkyl steht,
R⁶ für Wasserstoff, (C₁-C₆)-Alkyl oder für gegebenenfalls einfach bis dreifach, gleich oder verschieden durch Cyano, Nitro, Halogen, (C₁-C₆)-Alkyl, (C₁-C₆)-Alkoxy, (C₁-C₆)-Alkylthio, (C₁-C₄)-Halogenalkyl, (C₁-C₄)-Halogenalkoxy oder (C₁-C₄)-Halogenalkylthio mit jeweils 1 bis 5 Halogenatomen oder Hydroxy substituiertes Phenyl oder für durch Cyano, Nitro, Halogen, (C₁-C₆)-Alkyl, (C₁-C₆)-Alkoxy, (C₁-C₆)-Alkylthio, (C₁-C₄)-Halogenalkyl, (C₁-C₄)-Halogenalkoxy oder (C₁-C₄)-Halogenalkylthio mit jeweils 1 bis 5 Halogenatomen substituiertes Pyridyl steht und
R⁷ für (C₁-C₆)-Alkyl, (C₂-C₆)-Alkenyl, (C₂-C₆)-Alkinyl, Formyl, (C₁-C₆)-Alkyl-carbonyl, (C₁-C₆)-Halogenalkylcarbonyl mit 1 bis 6 Halogenatomen oder (C₁-C₆)-Alkoxycarbonyl steht,
Ar für jeweils gegebenenfalls einfach bis dreifach, gleich oder verschieden durch Halogen, (C₁-C₆)-Halogenalkyl, (C₁-C₆)-Halogenalkylthio, (C₁-C₆)-Halogenalkoxy, (C₁-C₆)-Alkoxy, Hydrazino, (C₁-C₆)-Dialkylhydrazino, Amino, (C₁-C₆)-Alkylamino, Di(C₁-C₆)-alkylamino, (C₁-C₆)-Alkylimino, Cyano, (C₁-C₆)-Alkylthio oder die Gruppierung 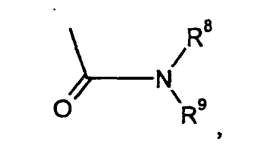 worin
R⁸ und R⁹ gleich oder verschieden sind und für Wasserstoff oder (C₁-C₆)-Alkyl stehen,
substituiertes Phenyl oder Pyridyl steht und
n für eine Zahl 0, 1 oder 2 steht.

2. Verbindungen gemäß Anspruch 1, **dadurch gekennzeichnet, dass**
R² für (C₁-C₄)-Halogenalkyl mit 1 bis 9 gleichen oder verschiedenen Halogenatomen aus der Reihe Fluor, Chlor und Brom, (C₂-C₄)-Halogenalkenyl mit 1 bis 5 gleichen oder verschiedenen Halogenatomen aus der Reihe Fluor, Chlor und Brom oder (C₂-C₄)-Halogenalkinyl mit 1 bis 5 gleichen oder verschiedenen Halogenatomen aus der Reihe Fluor, Chlor und Brom steht,
R⁴ für (C₁-C₄)-Alkyl, (C₁-C₄)-Halogenalkyl mit 1-3 Halogenatomen, (C₁-C₄)-Alkoxy-(C₁-C₂)-alkyl oder für gegebenenfalls einfach bis dreifach gleich oder verschieden durch Hydroxy, Cyano, Nitro, Halogen, (C₁-C₄)-Alkyl, (C₁-C₄)-Alkoxy, (C₁-C₂)-Halogenalkyl, (C₁-C₂)-Halogenalkoxy oder (C₁-C₂)-Halogenalkylthio mit jeweils 1 bis 3 Halogenatomen substituiertes Phenyl steht,
R⁵ für Wasserstoff oder (C₁-C₄)-Alkyl steht,
R⁶ für Wasserstoff, (C₁-C₄)-Alkyl oder für gegebenenfalls einfach oder zweifach, gleich oder verschieden durch Hydroxy, Cyano, Nitro, Halogen, (C₁-C₄)-Alkyl, (C₁-C₄)-Alkoxy, (C₁-C₂)-Halogenalkyl, (C₁-C₂)-Halogenalkoxy oder (C₁-C₂)-Halogenalkylthio mit jeweils 1 bis 3 Halogenatomen substituiertes Phenyl, insbesondere für 4-Hydroxy-3-methoxy-phenyl steht,
R⁷ für (C₁-C₄)-Alkyl, (C₂-C₄)-Alkenyl, (C₂-C₄)-Alkinyl, Formyl, (C₁-C₄)-Alkyl-carbonyl, (C₁-C₄)-Halogenalkyl-carbonyl mit 1 bis 5 gleichen oder verschiedenen Halogenatomen aus der Reihe Fluor, Chlor oder Brom oder (C₁-C₄)-Alkoxycarbonyl steht,
Ar für jeweils gegebenenfalls einfach bis dreifach, gleich oder verschieden durch Fluor, Chlor, Trifluormethyl, Trifluormethylthio, Trifluormethoxy, Methoxy, Hydrazino, Dimethylhydrazino, Amino, Methylamine, Dimethylamino, Iminomethyl, Cyano, Methylthio oder die Gruppierung 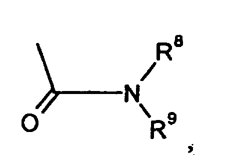 worin
R⁸ und R⁹ gleich oder verschieden sind und für Wasserstoff oder (C₁-C₄)-Alkyl stehen,
substituiertes Phenyl oder Pyridyl steht.

3. Verbindungen gemäß Anspruch 1 oder 2, **dadurch gekennzeichnet, dass**
R² für einen der folgenden Reste steht:
-CF₃,-CHF₂
-CF₂-CH₃, -CF₂-CHF₂, -CF₂-CHFCl,
-CH₂-CF₃, -CH₂-CF₂Cl,
-CH₂-CF₂-CHF₂,
-CF₂-CFCl-CF₃,
-C(Cl)(CF₃)-CF₂Cl, -C(Cl)(CF₃)-CHCl-CF₃,
-C(CF3)=CCl₂,
R³ für Wasserstoff, Amino oder eine der folgenden Gruppierungen steht:
-NH-CO-CH₃, -NH-CO-C₂H₅,
-N=CH-NH₂, -N=C(CH₃)-NH₂,
-N=CH-N(CH₃)₂, -N=C(CH₃)-N(CH₃)₂, 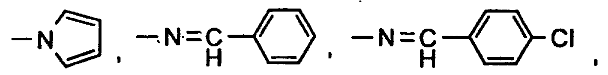 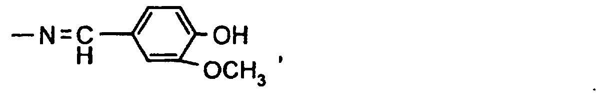
-NHC₂H₅ oder -NH-CH₂-CH=CH₂,
Ar für
(1) zweifach oder dreifach; gleich oder verschieden substituiertes Phenyl, wobei in der 2-Position Fluor oder Chlor, in der 4-Position Trifluormethyl und in der 6-Position Fluor, Chlor, Cyano, Methoxy, Methylthio, Trifluormethyl, Trifluormethoxy, Trifluormethylthio oder Hydrazino stehen; oder
(2) einen 2-Pyridyl-Rest, welcher in der 4-Position durch Trifluormethyl und in der 6-Position durch Fluor oder Chlor substituiert ist.

4. Verfahren zur Herstellung von Verbindungen der Formel (I) gemäß einem der Ansprüche 1 bis 3, **dadurch gekennzeichnet, dass** man
a) 3-Cyanopyrazol-Derivate der Formel (II), 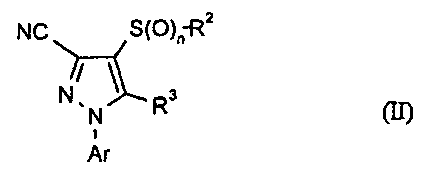 in welcher
Ar, R², R³ und n die in einem der Ansprüche 1 bis 3 angegebene Bedeutung haben,
mit Schwefelwasserstoff, gegebenenfalls in Gegenwart eines Reaktionshilfsmittels und gegebenenfalls in Gegenwart eines Verdünnungsmittels umsetzt;
oder
b) 3-Thiocarbamoylpyrazol-Derivate der Formel (III), 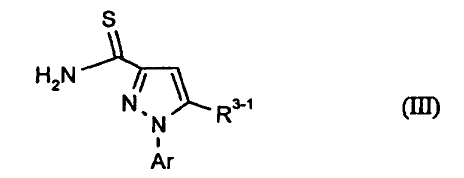 in welcher
Ar die oben angegebene Bedeutung hat und
R³⁻¹ für eine der folgenden Gruppierungen steht: 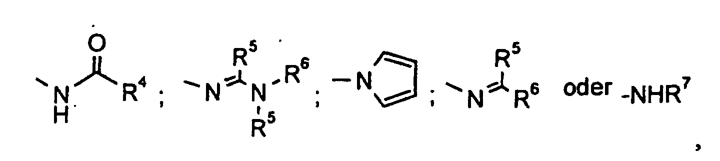 wobei
R⁴, R⁵, R⁶ und R⁷ die in Anspruch 1 oder 2 angegebene Bedeutung haben,
mit Sulfenylhalogeniden der Formel (IV),
Hal-S-R² (IV)
in welcher
R² die oben angegebene Bedeutung hat und
Hal für Halogen steht,
gegebenenfalls in Gegenwart eines Verdünnungsmittels und gegebenenfalls in Gegenwart eines Reaktionshilfsmittels umsetzt;
oder
c) die gemäß den Verfahren (a) oder (b) erhältlichen 2-Thiocarbamoylpyrazol-Derivate der Formel (Ia), 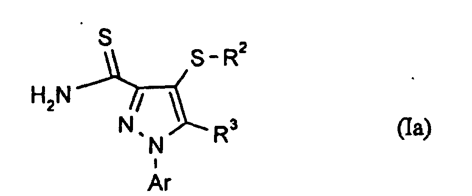
in welcher
Ar, R² und R³ die oben angegebene Bedeutung haben,
mit Oxidationsmitteln gegebenenfalls in Gegenwart eines Verdünnungsmittels und gegebenenfalls in Gegenwart eines Katalysators oxidiert.

5. Verbindungen der Formel (III), 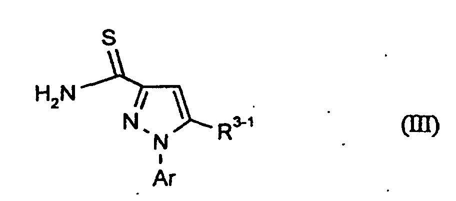 in welcher
Ar die in einem der Ansprüche 1 bis 3 angegebene Bedeutung hat und
R³⁻¹ für eine der folgenden Gruppierungen steht: 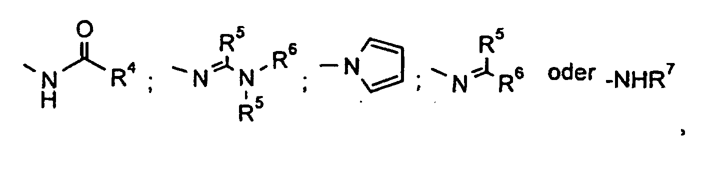 wobei
R4, R5, R⁶ und R⁷ die in Anspruch 1 oder 2 angegebene Bedeutung haben.

6. Verbindungen der Formel (VII), 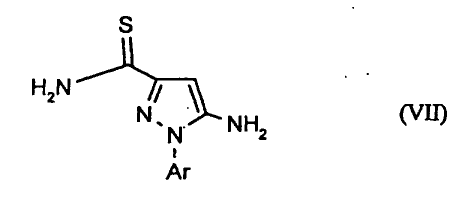 in welcher
Ar die in einem der Ansprüche 1 bis 3 angegebene Bedeutung hat.

7. Schädlingsbekämpfungsmittel, **gekennzeichnet durch** einen Gehalt an mindestens einer Verbindung gemäß einem der Ansprüche 1 bis 3.

8. Verwendung von Verbindungen gemäß einem der Ansprüche 1 bis 3 zur Bekämpfung von Schädlingen.

9. Verfahren zur Bekämpfung von Schädlingen, **dadurch gekennzeichnet, dass** man Verbindungen der Formel (I) gemäß einem der Ansprüche 1 bis 3 auf Schädlinge und/oder ihren Lebensraum einwirken lässt.

## Claims

1. Compounds of the general formula (I) 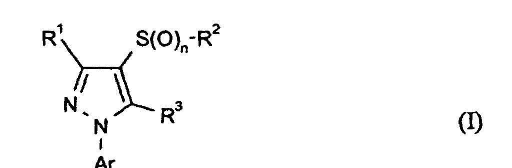 in which
R¹ represents H₂N-CS-,
R² represents (C₁-C₆)-halogenoalkyl having 1 to 12 halogen atoms; (C₂-C₆)-halogenoalkenyl having 1 to 8 halogen atoms or (C₂-C₆)-halogenoalkinyl having 1 to 6 halogen atoms,
R³ represents hydrogen, amino or one of the following groups: 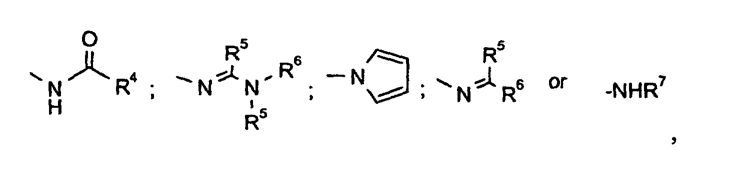 where
R⁴ represents (C₁-C₆)-alkyl, (C₁-C₆)-halogenoalkyl having 1 to 3 halogen atoms, (C₁-C₆)-alkoxy-(C₁-C₆)-alkyl, or represents phenyl or pyridyl, each of which is optionally monosubstituted to trisubstituted by identical or different substituents from the series cyano, nitro, halogen, (C₁-C₆)-alkyl (C₁-C₆)-alkoxy, (C₁-C₆)-alkylthio, (C₁-C₄)-halogenoalkyl, (C₁-C₄)-halogenoalkoxy, or (C₁-C₄)-halogenoalkylthio having in each case 1 to 5 halogen atoms,
R⁵ represents hydrogen or (C₁-C₆)-alkyl,
R⁶ represents hydrogen, (C₁-C₆)-alkyl, or phenyl which is optionally monosubstituted to trisubstituted by identical or different substituents from the series cyano, nitro, halogen, (C₁-C₆)-alkyl, (C₁-C₆)-alkoxy, (C₁-C₆)-alkylthio, (C₁-C₄)-halogenoalkyl, (C₁-C₄)-halogenoalkoxy or (C₁-C₄)-halogenoalkylthio having in each case 1 to 5 halogen atoms or by hydroxyl, or represents pyridyl which is substituted by cyano, nitro, halogen, (C₁-C₆)-alkyl, (C₁-C₆)-alkoxy, (C₁-C₆)-alkylthio, (C₁-C₄)-halogenoalkyl, (C₁-C₄)-halogenoalkoxy or (C₁-C₄)-halogenoalkylthio having in each case 1 to 5 halogen atoms, and
R⁷ represents (C₁-C₆)-alkyl, (C₂-C₆)-alkenyl, (C₂-C₆)-alkinyl, formyl, (C₁-C₆)-alkylcarbonyl, (C₂-C₆)-halogenoalkylcarbonyl having 1 to 6 halogen atoms or (C₁-C₆)-alkoxycarbonyl;
Ar represents in each case phenyl or pyridyl, each of which is optionally monosubstituted to trisubstituted by identical or different substituents from the series halogen, (C₁-C₆)-halogenoalkyl, (C₁-C₆)-halogenoalkylthio, (C₁-C₆)-halogenoalkoxy, (C₁-C₆)-alkoxy, hydrazino, (C₁-C₆)-dialkylhydrazino, amino, (C₁-C₆)-alkylamino, di(C₁-C₆)-alkylamino, (C₁-C₆)-alkylimino, cyano, (C₁-C₆)-alkylthio or the group 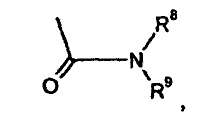 in which
R⁸ and R⁹ are identical or different and represent hydrogen or (C₁-C₆)-alkyl,
and
n represents a number 0, 1 or 2.

2. Compounds according to Claim 1, **characterized in that**
R² represents (C₁-C₄)-halogenoalkyl having 1 to 9 identical or different halogen atoms from the series fluorine, chlorine and bromine, (C₂-C₄)-halogenoalkenyl having 1 to 5 identical or different halogen atoms from the series fluorine, chlorine and bromine or (C₂-C₄)-halogenoalkinyl having 1 to 5 identical or different halogen atoms from the series fluorine, chlorine and bromine,
R⁴ represents (C₁-C₄)-alkyl, (C₁-C₄)-halogenoalkyl having 1-3 halogen atoms, (C₁-C₄)-alkoxy-(C₁-C₂)-alkyl, or represents phenyl which is optionally monosubstituted to trisubstituted by identical or different substituents from the series hydroxyl, cyano, nitro, halogen, (C₁-C₄)-alkyl, (C₁-C₄)-alkoxy, (C₁-C₂)-halogenoalkyl, (C₁-C₂)-halogenoalkoxy or (C₁-C₂)-halogenoalkylthio having in each case 1 to 3 halogen atoms,
R⁵ represents hydrogen or (C₁-C₄)-alkyl,
R⁶ represents hydrogen, (C₁-C₄)-alkyl, or represents phenyl which is optionally monosubstituted or disubstituted by identical or different substituents from the series hydroxyl, cyano, nitro, halogen, (C₁-C₄)-alkyl, (C₁-C₄)-alkoxy, (C₁-C₂)-halogenoalkyl, (C₁-C₂)-halogenoalkoxy or (C₁-C₂)-halogenoalkylthio having in each case 1 to 3 halogen atoms, in particular 4-hydroxy-3-methoxyphenyl,
R⁷ represents (C₁-C₄)-alkyl, (C₂-C₄)-alkenyl, (C₂-C₄)-alkinyl, formyl, (C₁-C₄)-alkylcarbonyl, (C₁-C₄)-halogenoalkylcarbonyl having 1 to 5 identical or different halogen atoms from the series fluorine, chlorine or bromine, or (C₁-C₄)-alkoxycarbonyl,
Ar represents phenyl or pyridyl, each of each is optionally monosubstituted to trisubstituted by identical or different substituents from the series fluorine, chlorine, trifluoromethyl, trifluoromethylthio, trifluoromethoxy, methoxy, hydrazino, dimethylhydrazino, amino, methylamino, dimethylamino, iminomethyl, cyano, methylthio or the group 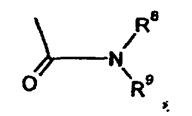
in which
R⁸ and R⁹ are identical or different and represent hydrogen or (C₁-C₄)-alkyl.

3. Compounds according to Claim 1 or 2, **characterized in that**
R² represents one of the following radicals:
-CF₃, -CHF₂
-CF₂-CH₃, -CF₂-CHF₂, -CF₂-CHFCl,
-CH₂-CF₃, -CH₂-CF₂Cl,
-CH₂-CF₂-CHF₂,
-CF₂-CFCl-CF₃,
-C(Cl)(CF₃)-CF₂Cl, -C(Cl)(CF₃)-CHCl-CF₃,
-C(CF3)=CCl₂,
R³ represents hydrogen, amino or one of the following groups:
-NH-CO-CH₃, -NH-CO-C₂H₅,
-N=CH-NH₂, -N=C(CH₃)-NH₂'
-N=CH-N(CH₃)₂, -N=C(CH₃)-N(CH₃)₂, 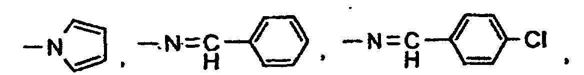 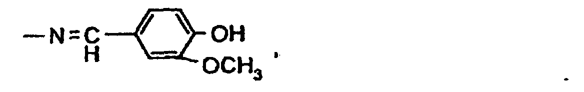
-NHC₂H₅ or -NH-CH₂-CH=CH₂,
Ar represents
(1) phenyl which is disubstituted or trisubstituted by identical or different substituents, where the 2-position is occupied by fluorine or chlorine, the 4-position by trifluoromethyl and the 6-position by fluorine, chlorine, cyano, methoxy, methylthio, trifluoromethyl, trifluoromethoxy, trifluoromethylthio or hydrazino; or
(2) a 2-pyridyl radical which is substituted in the 4-position by trifluoromethyl and in the 6-position by fluorine or chlorine.

4. Process for the preparation of compounds of the formula (I) according to one of Claims 1 to 3, **characterized in that**
a) 3-cyanopyrazole derivatives of the formula (II) 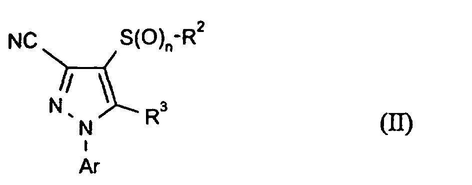 in which
Ar, R², R³ and n are as defined in one of Claims 1 to 3
are reacted with hydrogen sulphide, if appropriate in the presence of a reaction auxiliary and if appropriate in the presence of a diluent;
or
b) 3-thiocarbamoylpyrazole derivatives of the formula (III) 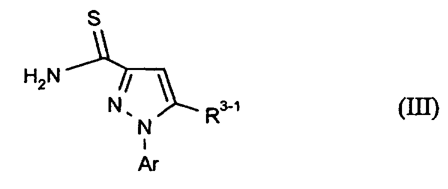 in which
Ar is as defined above and
R³⁻¹ represents one of the following groups: 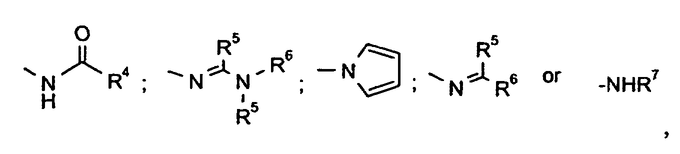
where
R⁴, R⁵, R⁶ and R⁷ are as defined in Claim 1 or 2
are reacted with sulphenyl halides of the formula (IV)
Hal-S-R² (IV)
in which
R² is as defined above and
Hal represents halogen,
if appropriate in the presence of a diluent and if appropriate in the presence of a reaction auxiliary;
or
c) the 2-thiocarbamoylpyrazole derivatives of the formula (Ia) 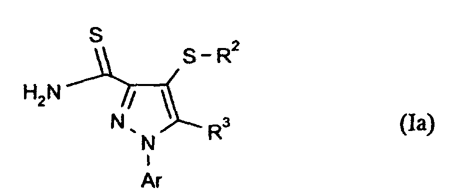
in which
Ar, R² and R³ are as defined above and which can be obtained by process (a) or (b)
are oxidized with oxidants, if appropriate in the presence of a diluent and if appropriate in the presence of a catalyst.

5. Compounds of the formula (III) 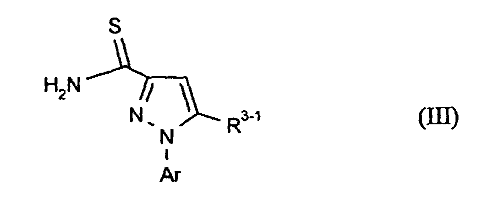 in which
Ar is as defined in one of Claims 1 to 3 and
R³⁻¹ represents one of the following groups: 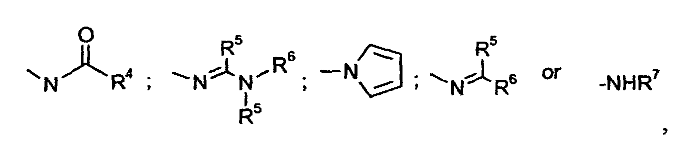 where
R⁴, R⁵, R⁶ and R⁷ are as defined in Claim 1 or 2.

6. Compounds of the formula (VII) 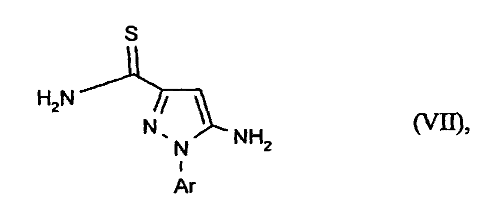 in which
Ar is as defined in one of Claims 1 to 3.

7. Pesticides, **characterized in that** they comprise at least one compound according to one of Claims 1 to 3.

8. Use of compounds according to one of Claims 1 to 3 for controlling pests.

9. Method of controlling pests, **characterized in that** compounds of the formula (I) according to one of Claims 1 to 3 are allowed to act on pests and/or their environment.

## Revendications

1. Composés de la formule générale (I) : 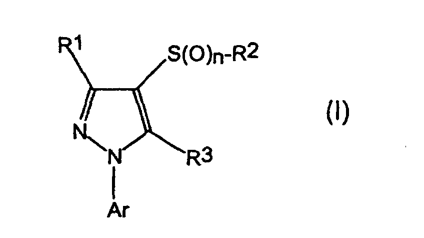 dans laquelle :
R¹ représente H₂N-CS-;
R² représente un radical halogénoalcoyle (C₁-C₆) ayant 1 à 12 atomes d'halogène ; un radical halogénoalcényle (C₂-C₆) ayant 1 à 8 atomes d'halogène ou un radical halogénoalcynyle (C₂-C₆) ayant 1 à 6 atomes d'halogène ;
R³ représente l'atome d'hydrogène, le radical amino ou un des groupements suivants : 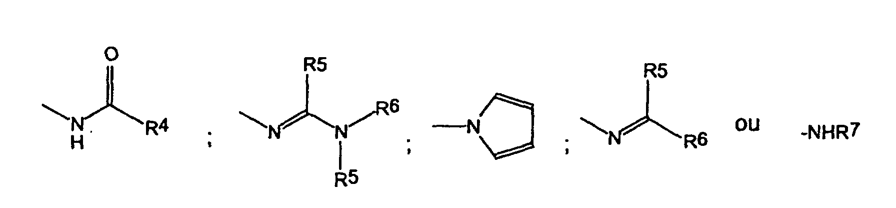 où
R⁴ représente un radical alcoyle (C₁-C₆), halogénoalcoyle (C₁-C₆) ayant 1 à 3 atomes d'halogène, alcoxy (C₁-C₆) -alcoyle (C₁-C₆) ou le radical phényle ou pyridyle, chaque fois le cas échéant substitué une à trois fois, de manière identique ou différente, par le radical cyano, nitro, un atome d'halogène, le radical alcoyle (C₁-C₆), alcoxy (C₁-C₆), alcoylthio (C₁-C₆), halogénoalcoyle (C₁-C₄), halogénoalcoxy (C₁-C₄) ou halogénoalcoylthio (C₁-C₄) ayant chaque fois 1 à 5 atomes d'halogène,
R⁵ représente l'atome d'hydrogène ou un radical alcoyle (C₁-C₆),
R⁶ représente l'atome d'hydrogène, un radical alcoyle (C₁-C₆), ou représente le radical phényle le cas échéant substitué une à trois fois, de manière identique ou différente, par le radical cyano, nitro, un atome d'halogène, le radical alcoyle (C₁-C₆), alcoxy (C₁-C₆), alcoylthio (C₁-C₆), halogénoalcoyle (C₁-C₄) , halogénoalcoxy (C₁-C₄) ou halogénoalcoylthio (C₁-C₄) ayant chaque fois 1 à 5 atomes d'halogène ou le radical hydroxyle, ou représente le radical pyridyle le cas échéant substitué une à trois fois, de manière identique ou différente, par le radical cyano, nitro, un atome d'halogène, le radical alcoyle (C₁-C₆), alcoxy (C₁-C₆), alcoylthio (C₁-C₆), halogénoalcoyle (C₁-C₄), halogénoalcoxy (C₁-C₄) ou halogénoalcoylthio (C₁-C₄) ayant chaque fois 1 à 5 atomes d'halogène, et
R⁷ représente un radical alcoyle (C₁-C₆), alcényle (C₂-C₆) , alcynyle (C₂-C₆), formyle, alcoyl (C₁-C₆)-carbonyle, halogénoalcoyl (C₁-C₆)-carbonyle ayant 1 à 6 atomes d'halogène ou alcoxy (C₁-C₆)-carbonyle,
Ar représente le radical phényle ou pyridyle chaque fois le cas échéant substitué une à trois fois, de manière identique ou différente, par un atome d'halogène, un radical halogénoalcoyle (C₁-C₆), halogénoalcoylthio (C₁-C₆), halogénoalcoxy (C₁-C₆), alcoxy (C₁-C₆), hydrazino, dialcoyl (C₁-C₆)-hydrazino, amino, alcoylamino (C₁-C₆), dialcoyl (C₁-C₆)-amino, alcoylimino (C₁-C₆), cyano, alcoylthio (C₁-C₆) ou par le groupement : 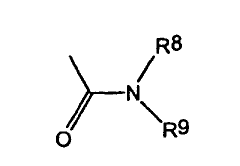 où
R⁸ et R⁹ sont identiques ou différents et représentent l'atome d'hydrogène ou un radical alcoyle (C₁-C₆), et
n représente le nombre 0, 1 ou 2.

2. Composés selon la revendication 1, **caractérisés en ce que**
R² représente un radical halogénoalcoyle (C₁-C₄) ayant 1 à 9 atomes d'halogène identiques ou différents, de la série des atomes de fluor, de chlore et de brome ; un radical halogénoalcényle (C₂-C₄) ayant 1 à 5 atomes d'halogène identiques ou différents, de la série des atomes de fluor, de chlore et de brome ou un radical halogénoalcynyle (C₂-C₄) ayant 1 à 5 atomes d'halogène identiques ou différents, de la série des atomes de fluor, de chlore et de brome ;
R⁴ représente un radical alcoyle (C₁-C₄), halogénoalcoyle (C₁-C₄) ayant 1 à 3 atomes d'halogène, alcoxy (C₁-C₄)-alcoyle (C₁-C₂) ou le radical phényle le cas échéant substitué une à trois fois, de manière identique ou différente, par le radical hyroxyle, cyano, nitro, un atome d'halogène, le radical alcoyle (C₁-C₄), alcoxy (C₁-C₄), halogénoalcoyle (C₁-C₂), halogénoalcoxy (C₁-C₂) ou halogénoalcoylthio (C₁-C₂) ayant chaque fois 1 à 3 atomes d'halogène,
R⁵ représente l'atome d'hydrogène ou un radical alcoyle (C₁-C₄),
R⁶ représente l'atome d'hydrogène, un radical alcoyle (C₁-C₄), ou représente le radical phényle le cas échéant substitué une ou deux fois, de manière identique ou différente, par le radical hydroxyle, cyano, nitro, un atome d'halogène, le radical alcoyle (C₁-C₄), alcoxy (C₁-C₄), halogénoalcoyle (C₁-C₂), halogénoalcoxy (C₁-C₂) ou halogénoalcoylthio (C₁-C₂) ayant chaque fois 1 à 3 atomes d'halogène, en particulier le radical 4-hydroxy-3-méthoxyphényle,
R⁷ représente un radical alcoyle (C₁-C₄), alcényle (C₂-C₄), alcynyle (C₂-C₄), formyle, alcoyl (C₁-C₄)-carbonyle, halogénoalcoyl (C₁-C₄)-carbonyle ayant 1 à 5 atomes d'halogène identiques ou différents, de la série des atomes de fluor, de chlore et de brome ou alcoxy (C₁-C₄)-carbonyle,
Ar représente le radical phényle ou pyridyle chaque fois le cas échéant substitué une à trois fois, de manière identique ou différente, par l'atome de fluor, de chlore, le radical trifluorométhyle, trifluorométhoxy, méthoxy, hydrazino, diméthylhydrazino, amino, méthylamino, diméthylamino, iminométhyle, cyano, méthylthio ou par le groupement : 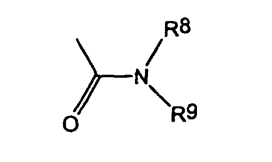 où
R⁸ et R⁹ sont identiques ou différents et représentent l'atome d'hydrogène ou un radical alcoyle (C₁-C₄).

3. Composés selon la revendication 1 ou 2, **caractérisés en ce que**
R² représente un des restes suivants :
-CF₃, -CHF₂,
-CF₂-CH₃, -CF₂-CHF₂, -CF₂-CHFCl,
-CH₂-CF₃, -CH₂-CF₂Cl,
-CH₂-CF₂-CHF₂,
-CF₂-CFCl-CF₃,
-C(Cl) (CF₃) -CF₂Cl, -C(Cl) (CF₃) -CHCl-CF₃,
-C (CF₃)=CCl₂,
R³ représente l'atome d'hydrogène, le radical amino ou un des groupements suivants :
-NH-CO-CH₃, -NH-CO-C₂H₅,
-N=CH-NH₂, -NH=C(CH₃)-NH₂,
-N=CH-N(CH₃)₂, -NH=C(CH₃)-N(CH₃)₂, 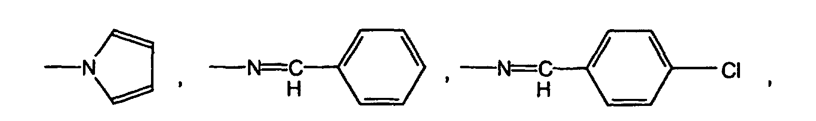 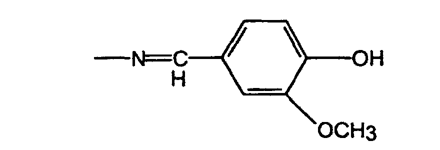
-NHC₂H₅ ou -NH-CH₂-CH=CH₂,
Ar représente:
(1) le radical phényle substitué deux fois ou trios fois, de manière identique ou différente, où en position 2, se trouve l'atome de fluor ou de chlore, en position 4, se trouve le radical trifluorométhyle et en position 6, se trouve l'atome de fluor ou de chlore, le radical cyano, méthoxy, méthylthio, trifluorométhyle, trifluorométhoxy, trifluorométhylthio ou hydrazino, ou
(2) un reste 2-pyridyle, qui est substitué en position 4 par le radical trifluorométhyle et en position 6 par l'atome de fluor ou de chlore.

4. Procédé de préparation des composés de la formule (I) suivant l'une quelconque des revendications 1 à 3, **caractérisé en ce que** :
a) on fait réagir un dérivé 3-cyanopyrazole de la formule (II) : 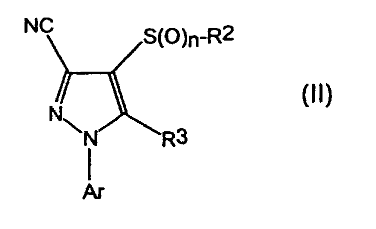 dans laquelle :
Ar, R², R³ et n ont la signification donnée dans l'une quelconque des revendications 1 à 3,
avec le sulfure d'hydrogène, le cas échéant en présence d'un auxiliaire de réaction et le cas échéant, en présence d'un agent de dilution, ou
b) on fait réagir un dérivé 3-thiocarbamoylpyrazole de la formule (III) : 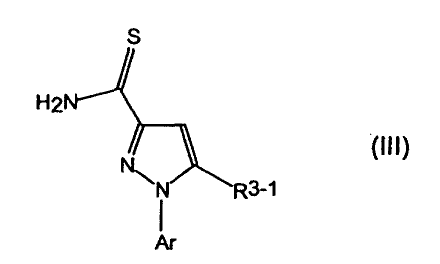 dans laquelle :
Ar a la signification donnée ci-dessus, et
R³⁻¹ représente un des groupements suivants : 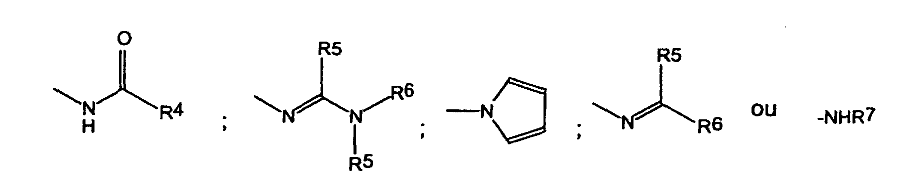
dans laquelle :
R⁴, R⁵, R⁶ et R⁷ ont la signification donnée dans la revendication 1 ou 2,
avec un halogénure de sulfényle de la formule (IV) :
Hal - S - R² (IV)
dans laquelle :
R² a la signification donnée ci-dessus, et
Hal représente un atome d'halogène,
le cas échéant en présence d'un agent de dilution et le cas échéant, en présence d'un auxiliaire de réaction, ou
c) on oxyde le dérivé 2-thiocarbamoylpyrazole de la formule (Ia), obtenu selon le procédé (a) ou (b) : 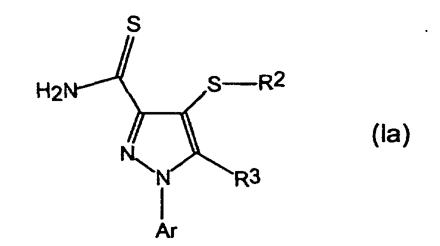
dans laquelle :
Ar, R² et R³ ont la signification donnée ci-dessus,
avec un agent d'oxydation, le cas échéant en présence d'un agent de dilution et le cas échéant, en présence d'un catalyseur.

5. Composés de la formule (III) : 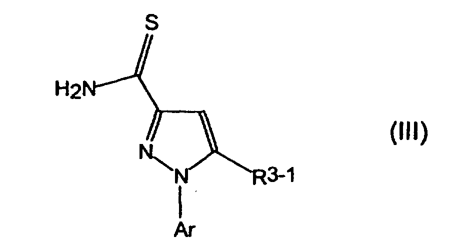 dans laquelle :
Ar a la signification donnée dans l'une quelconque des revendications 1 à 3, et
R³⁻¹ représente un des groupements suivants : 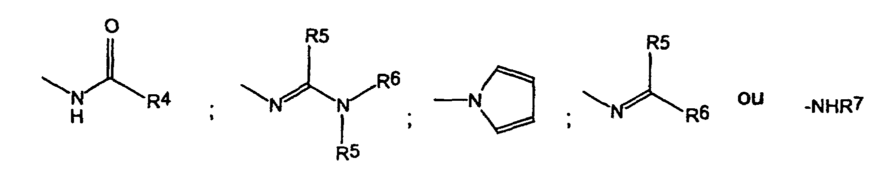
dans laquelle :
R⁴, R⁵, R⁶ et R⁷ ont la signification donnée dans la revendication 1 ou 2.

6. Composés de la formule (VII) : 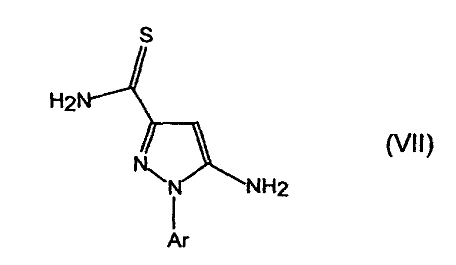 dans laquelle :
Ar a la signification donnée dans l'une quelconque des revendications 1 à 3.

7. Agent de lutte contre les parasites, **caractérisé par** une teneur en au moins un composé suivant l'une quelconque des revendications 1 à 3.

8. Utilisation de composés suivant l'une quelconque des revendications 1 à 3 pour lutter contre les parasites.

9. Procédé pour lutter contre les parasites, **caractérisé en ce que** l'on fait agir les composés de la formule (I) suivant l'une quelconque des revendications 1 à 3, sur les parasites et/ou leur biotope.
